# EUROPEAN PATENT SPECIFICATION

(11) **EP 3 813 912 B1**
(45) Date of publication and mention of the grant of the patent: **19.01.2022**
(21) Application number: 20734927.5
(22) Date of filing: 25.06.2020
(51) Int. Cl.: A61M 15/00, A61M 16/00

(54) **AN INHALER ASSEMBLY COMPRISING A DETACHABLE FLOW SENSOR**
INHALATIONSANORDNUNG MIT EINEM ABNEHMBAREN DURCHFLUSSSENSOR
ENSEMBLE D'INHALATEUR COMPRENANT UN CAPTEUR DE DÉBIT AMOVIBLE

(30) Priority: 04.07.2019 DK PA201970437
(43) Date of publication of application: 05.05.2021
(73) Proprietor: NuvoAir AB, 114 57 Stockholm (SE)
(72) Inventor: CONSOLI, Lorenzo, 114 52 Stockholm (SE); BOSAEUS, Linus, 114 18 Stockholm (SE); AIDEHAG, Fredrik, 117 56 Stockholm (SE)
(74) Representative: Inspicos P/S
(86) International application number: PCT/EP2020/067875
(87) International publication number: WO 2021/001260

(56) References cited:
- WO-A1-2017/178865
- US-A1- 2016 144 141
- US-A1- 2016 325 058

## Description

The present invention relates to an assembly of a medication dispenser, and in particular an inhaler, and a detachably attachable activation and flow sensor.

In medication dispensers, and not the least in inhalers, it is not uncommon to provide an activation detector for detecting that a medication dose has been ejected or made available. This information may be used for e.g. ascertaining compliance with a medication scheme.

Technology of this type may be seen in WO2017/178865, US2016/144141, and US2016/325058.

However, activation may take place without the dose having been administered. The present invention relates to a manner of obtaining with a larger probability information of whether a dose was actually administered correctly.

In a first aspect, the invention relates to an assembly according to claim 1.

In the present context, the medication inhaler may be any known type of inhaler, such as a metered dose inhaler (MDI), dry powder inhaler (DPI), soft mist inhaler (SMI). An inhaler may thus output any type of medication and on any form, mist, powder, gas, suspension or the like.

Inhalers have a flow channel into which the medication is made available and through which a flow is created by inhalation so that the medication is entrained in the flow and thereby brought to the lungs of the inhaling person. The flow is created in a flow channel which the user may engage (the lips or nose) to ensure that the medication is guided toward the lungs of the person. The flow channel is defined by portions of a housing of the inhaler.

The inhaler housing may comprise therein the medication or a container comprising the medication. Alternatively, the medication container or the like may be provided outside of the housing. Usually, the medication container or the like may be attached, detachably or not, to the housing.

In some embodiments, the medication is provided in a pressurized canister capable of outputting a metered dose upon longitudinal compression of the canister, usually by translating a stem thereof into or toward the main canister. The output of the stem will output the medication. The actual medication may be liquid or powder and mixed into a component with low evaporation point so that when ejected (and exposed to ambient pressure), a mist will be formed of the medication entrained in a gas.

In other embodiments, the medication is provided as a dry powder, such as under ambient pressure, either as a bulk amount from which doses are selected or as individual doses, such as in blister pack.

The flow channel usually has a single output, such as a mouth piece or opening engageable by the user. The flow channel may have one or more well defined inlets. In some situations, the housing is simply designed open so that a sufficient flow of air is possible into the flow channel.

Often, the flow channel is at least substantially closed between the medication dispensing opening and the output, so that no significant air flow is possible from the surroundings and into that portion of the flow channel, as this flow would increase the amount of air which has to be inhaled until the medication is delivered. If this amount of air is excessive, the user will not be able to have all medication delivered even when the flow is correct.

As mentioned, the medication dispensing opening may be the opening of a stem of a pressurized canister. In addition or alternatively, the medication dispensing opening may be an opening through which the medication, already entrained in an air flow, is guided into the flow channel. In this situation the only significant flow through this opening is seen when the medication is dispensed there through into the flow channel. When no medication is dispensed, the opening may be closed, such as in the form of a closed channel.

In one embodiment, the flow channel may, at least partly, extend through the medication dispensing opening. For example, if a powder is to be drawn from a blister, at least part of the flow may be directed through the blister to have the powder entrained in the air flow and ejected from the blister and into the remainder of the flow channel.

The activation element is manually operatable, such as depressible. The manual actuation, such as a force, may be applied directly to e.g. a canister or puncturing element for puncturing a blister. Alternatively, the manual actuation may generate a signal causing an actuation element, such as an actuator, to operate and then cause the release or ejection of the medication.

A manually operatable element may be a push button, rotatable element, lever, active area of a touch screen, key on a keyboard or the like.

The activation detector is capable of determining when the activation element has been activated. In one situation, the activation detector may be a sensitive element capable of determining when a force, such as a sufficient force, has been exerted to the activation element, such as a push button, rotatable element or lever, or the like. When the activation is mechanical, a force determination may be suitable.

Force sensitive elements may be strain gauges, push buttons, electronic circuits, for example. Also, acceleration sensors may be used for determining the force or activation in that a resistance may need to be overcome which may create a recognisable movement or shaking of the inhaler. When a blister pack is pierced, this will create a particular movement of the inhaler, as may the compression of a canister and/or the ejection of a jet in the flow channel.

If the activation is electrical, the activation detector may determine if and when the activation element outputs a suitable or sufficient signal.

The flow sensor may be based on any type of sensor, such as a pressure or vacuum sensor.

Clearly, the flow at the flow sensor may not be identical to that at other positions of the flow channel, such as at the medication dispensing opening or an output of the flow channel. However, a relation between the flow at the flow sensor and the flow at any desired position of the flow channel may easily be derived.

The sensing element is detachably attachable to the medication inhaler. In this manner, the sensing element may be sequentially connected to a number of inhalers, such as identical or similar inhalers. The attachment may be a snap fitting, a press fitting, engagement using a bayonet connection, clicking, dual sided tape, stickers, glue or the like.

The assembly further comprises a processor in communication with the flow sensor and the activation detector, the processor being configured to detect an inhalation when a flow is sensed and an activation is detected.

The processor may be any type of processor, such as a controller, ASIC, DSP, software programmable or not.

The processor is able to receive an output from the flow sensor and an output from the activation sensor. From the output of the flow sensor, an actual flow may be estimated or determined and/or it may be determined whether the flow is above or below a threshold value if desired.

From the output of the activation sensor, activation may be determined, for example. The activation sensor may output a signal identifying an activation and/or a signal relating to a force/torque with which the activation element has been engaged. Activation may then be assumed if the force exceeds a threshold value or is within a predetermined force/torque interval.

In addition, detector may determine a point in time of activation. Otherwise, the processor may determine this point in time.

Also, the sensor and/or processor may determine the flow over time, such as using the same clock or time as the processor or sensor for the activation.

In this manner, it may be determined which flow is present at or after the point in time of activation.

If the inhaler comprises a pressurized canister, the medication may be fed into the flow channel during a limited period of time immediately after activation, so that it is desired to know the flow during that time interval to determine whether the activation led to a correct inhalation. If the flow during this period of time was too low, too little medication may have reached the lungs but instead settled in the inhaler our mouth, whereas if the flow was too high, too much medication may have settled in the throat and not reached the lungs.

Actually, from the flow and activation point in time, the actual amount of medication reaching the lungs may be determined. Often, medication manufacturers provide information as to the optimum flow, as the optimal flow would depend on both droplet/particle size and also to a certain extent to the anatomy of the mouth and throat of the person.

If the inhaler comprises a powder under ambient pressure, the powder may remain in the same place if no flow is present. In that situation, it may be less important how long it takes from activation to inhalation. However, it may be desired to set a maximum allowable period of time between activation and (sufficient) inhalation, as the powder may be shaken out of the container or become humid and therefore un-inhalable. What may also be relevant is the flow provided, as this may be used for determining how much medication reached the lungs. Again, the flow over time may be determined, or it may be determined that the flow exceeds a minimum limit, for example.

Thus, in general, it may be desired to detect an inhalation, or even a correct or sufficient inhalation, when a period of time between the time of activation and when a sufficient flow (exceeding a threshold flow) is less than a predetermined period of time, which may be 10s or less, such as 8s or less, such as 5s or less, such as 3s or less such as 1s or less. It may be required that this flow exists already at activation.

In addition or alternatively, it may be desired to detect an inhalation, such as a correct or sufficient inhalation, if the flow, subsequent to activation, has been within a predetermined flow interval, such as for a period of time exceeding a threshold time, such as more than 1s, such as more than 2s, such as more than 3s, such as more than 4s, such as more than 5s. This flow may be required to take place within no more than 20s from activation, such as no more than 10s from activation, such as no more than 5s from inhalation.

In one embodiment, the medication inhaler comprises a compressible container with pressurized liquid/fluid/gas positioned in the housing, where the flow channel extends in the housing along the container and where the activation sensor is provided at one end of the container. This container may comprise a fluid under pressure as described above.

In this situation, the detector may be provided at the output end. If the container comprises a displaceable stem, the detector may be configured to detect displacement of the stem or container in relation to each other. In this situation, the stem may be fixed in relation to the inhaler housing so that this displacement may be detected as a displacement between the container and the inhaler housing.

Alternatively, the detector may be a force sensor configured to detect or determine a force with which the container is compressed.

The container may resist compression unless the force exceeds a threshold force. The detector may be configured to determine a compression by detecting a force exceeding this threshold force. Force or torque detectors may be e.g. strain gauges, push buttons or electronic circuits.

The sensing element comprises a portion extending from the activation detector and into the flow channel, a second flow channel extending from the flow sensor to the flow channel through the portion. In this situation, the flow sensor may be provided outside of the flow channel. In that situation, the second flow channel may allow the flow sensor to estimate the flow in the flow channel. In this manner, the sensor and the detector may be positioned close to each other. Also, the second flow channel may be formed in a portion of the sensing element extending into the flow channel. This portion may be made rather thin so as to disturb the flow in the flow channel as little as possible.

The extending portion may have an opening into the second flow channel, the opening being positioned at least a minimum depth into the channel, such as at least 1mm, at least 2mm, at least 3mm or more.

It is preferred that the activation detector and flow sensor are provided in the same element and attached to each other, such as provided in the same housing. This housing may be resilient or compliant if desired.

In one embodiment, the sensing element comprises a sensor housing portion configured to engage the inhaler housing and form a part of the flow channel, the flow sensor being provided in the sensor housing. In this embodiment, the flow channel already existing in the inhaler housing may be extended by the sensor housing portion. The flow channel may thus also be defined between the sensor housing portion and a portion of the inhaler housing and/or a medication container. In one situation, the sensor housing is attached to the inhaler and extends the inhaler housing to extend the flow channel existing also between a medication container and the inhaler housing.

Preferably, the processor forms part of the sensing element. In this manner, the sensing element may be a self-contained unit. The sensing element may further comprise a power source, such as a battery, storage, a clock and/or communication capabilities for e.g. receiving data, such as calibration data and/or algorithms for use in the sensing/detection/determination and/or for outputting information relating to the sensed flow, detected activation(s) and/or inhalations.

Communication may be wireless or wired. If wired communication is used, power or charging may also be provided by that cable. Wireless communication may be on any protocol, such as Bluetooth, ZWave, LoRa, or the like. Also, radio communication, WiFi or optical communication may be used.

The storage may be used for holding calibration data, software programs, sensed/detected or determined/calculated data or information.

Naturally, information may be fed to the storage such as the type of medication of the inhaler as well as the identity of the user and/or a medication scheme. Adherence to the medication scheme may then be determined.

The sensing element may also comprise additional sensors, such as accelerometers or the like. In this manner, operation of one of the sensors may waken the processor so as to be ready for receiving signals from the sensor and detector. The activation of the sensor may also wake up the sensor or detector if required.

As mentioned, the sensor element is detachably attachable to an inhaler in order to be used sequentially with different inhalers. The inhalers may be identical or not.

A method of using the assembly may comprise detachably attaching the sensor element to the medication inhaler. This would mean attaching using e.g. one of the attachment methods and means described above. Due to this attachment, the portion may be positioned in the flow channel and the activation detector is positioned in a desired position.

Subsequently to attachment, the inhaler may be used as intended. Thus, activation may take place.

Activating the inhaler, so as to dispense a dose of medication through the medication dispensing opening, preferably causes the activation detector to detect the activation. The detector then may output a signal to the processor.

During or after the activation, a flow may be provided in the flow channel. The flow sensor then detects the flow in the flow channel. This detection may be a detection of the actual flow, such as over time, or a determination that the flow is within a predetermined flow interval, for example.

A signal is forwarded from the activation detector and a signal is forwarded from the flow sensor to the processor, where after the processor may determine whether an inhalation, such as a correct or sufficient inhalation, has taken place.

As mentioned above, the determination of whether there has been an inhalation and optionally whether it was correct or sufficient may be made in a number of manners. Often, the determining step comprises the processor determining whether a correct inhalation has taken place based on a period of time from activation of the activation detector and until a flow is detected exceeding a threshold value. In addition or alternatively, a period of time in which the flow is within a predetermined interval may be taken into account.

In one situation, the determining step comprises the processor determining whether a correct inhalation has taken place based on a flow in the flow channel at a point in time of activation of the activation detector. This may be the situation, as described above, when the inhaler comprises a pressurized canister.

Clearly, additional information may be used in the determination, such as information about the drug, particle/droplet size, flow parameters (for pressurized canisters) and the like. Different particle/droplet sizes require different flows to reach the lungs. The additional flow (volume and velocity) of the dose ejected from a pressurized canister may be taken into account when determining the flow, such as at an output of the inhaler. For powder inhalers, the flow required to remove the powder from the container may be taken into account as may the time required to remove all or a sufficient portion of the powder.

In one embodiment, the method further comprises the step of removing the sensor element from the medication inhaler and attaching the sensor element to another medication inhaler. The removal may comprise removing the portion from the flow channel of the first inhaler and providing in it the flow channel of the other inhaler. The other medication inhaler may be identical to or similar to the first inhaler. A similar inhaler may have a housing of the same shape and a medication container of the same type. After attachment to the other medication inhaler, this inhaler may be activated as the first one. It may be desired to enter information or data relating to the other inhaler or to the fact that inhaler has been replaced into the sensing element.

In the following, preferred embodiments will be described with reference to the drawing, wherein:
- Figure 1 illustrates a first embodiment of an assembly, disassembled, according to the invention,
- Figure 2 illustrates the assembly of figure 1 in the assembled state,
- Figure 3 illustrates another embodiment of an assembly according to the invention,
- Figure 4 illustrates the sensing element of the assembly in figure 3.

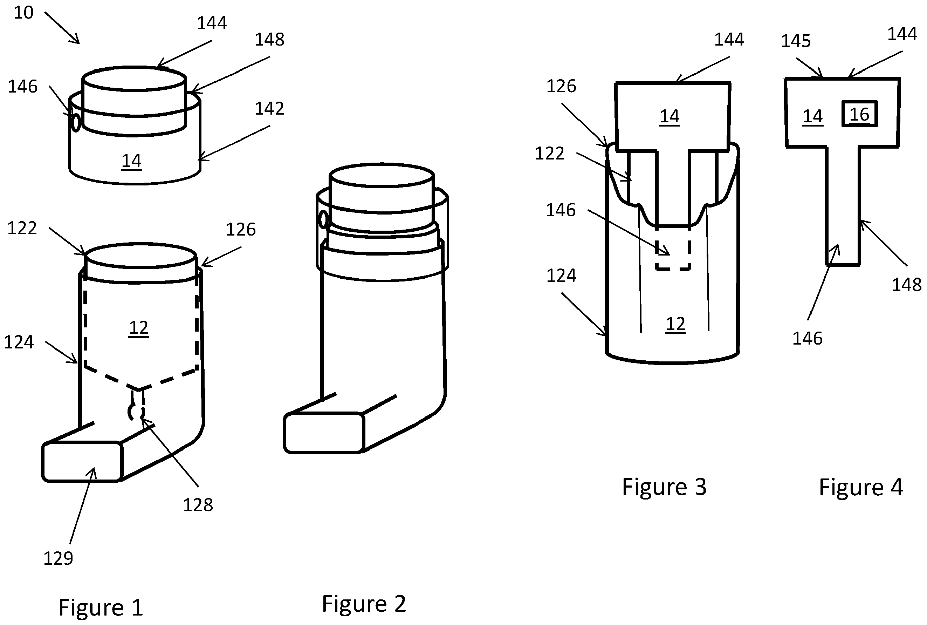

In figure 1, an assembly 10 is illustrated having a sensing element 14 and a medical inhaler 12.

The inhaler 12 is a standard PMDI (Pressurized Metered Dose Inhaler) having an inhaler housing 124 in which a pressurized canister 122 is provided. Between the canister and the housing, a flow channel 126 is defined which extends from a top of the housing 124, along the canister, past a medication dose output 128 and to an outlet or mouthpiece 129.

The usual operation of a PMDI is that a user will inhale through the mouthpiece 129 and thereby create an air flow in the channel 126. When this flow is created, the user will compress the canister by pushing the top and bottom of the PMDI so that the canister is compressed longitudinally, whereby an ejection stud is forced into the canister and thus causes the emission of a metered dose of the contents of the canister. This dose is emitted as droplets or a mist from the output 128 into the flow and thus carried by the flow to the user's lungs.

The operation of the PMDI thus is the compression of the canister.

However, it is desired to ensure that the user takes the medication correctly. A compression of the canister may take place by accident, such as if the PMDI is in a pocket of the person or during testing or priming of the device. Also, the person may compress the canister while there is no flow or an incorrect flow in the flow channel so that the dose output does not - or not to a desired degree - reach the lungs of the person. The flow carrying the medication should be within a predetermined flow interval in order for the medication (droplets, powder) to negotiate the throat of the person and reach the lungs. A too low flow will allow large portions of the medication to settle in the flow channel, the mouth piece and the person's mouth. A too high flow will make the medication unable to negotiate the bend of the throat and thus deposit in the throat of the person.

The assembly 10 therefore comprises the sensor element 14 having an activation detector 144 and a housing or collar 142 fitting outside of the housing 124 and inside which the activation detector 144 is provided and which also, between the activation detector 144 and the housing 142 defines an extension of the flow channel 126.

In figure 2, the assembled assembly is seen.

Inside the flow channel extension 148, a flow sensor 146 is provided.

Thus, from the activation sensor, which may be a pressure gauge, it may be determined when a force is applied which is sufficient to compress the canister and thus emit a dose. Also, the flow sensor may determine the flow at any point in time, such as at or around the point in time of activation.

From this information, it may be determined whether a dose has been emitted and whether it can be assumed to have been correctly delivered to a person.

Clearly, this type of assembly may use other types of medication delivery than pressurized canisters. Medication is also seen as powders in capsules which are also provided in inhalers. Here, the activation is the puncturing of the capsule, where the flow goes through the capsule to entrain the medication in the air flow towards a mouthpiece and thus the person's lungs.

In this respect, the medication may not be lost if the capsule is punctured without there being any flow. If no flow is present, the medication, or parts thereof, may remain in the capsule at least for a period of time.

Thus, depending on the type of medication dispensing, higher or lower requirements may be put on the timing between activation and inhalation.

However, from the data from the activation sensor and the flow sensor, it may be estimated either how much medication reached the person's lungs and/or whether a correct or sufficient inhalation or medication has taken place.

The advantage of the assembly structure is that the sensor element 14 may be re-used when an inhaler or a canister needs replacing. Thus, the sensor element 14 preferably is detachably attached to the inhaler, such as by press-fitting, a bayonet coupling, clicking, or the like. Then, the sensor element may comprise more expensive elements and may thus provide a standard, purely mechanical inhaler with a lot of relevant capabilities.

Naturally, the sensor element may comprise a controller for receiving the output from the activation sensor and the flow sensor. The controller may have a clock for emitting points in time, memory for storing data, additional sensors, and communication capabilities to other elements such as mobile phones, pads, computers, the internet, the telephone network, a server or the like. Thus, the sensor element may comprise means for wired or wireless communication (GSM, Bluetooth, WiFi, ZWave, Zigbee, LoRa, Ethernet, Firewire, USB, AppleTalk or the like).

In figure 3, another assembly is illustrated again with a PMDI having a housing 124 in which a canister 122 is present and again forming a flow channel 126. In this embodiment, the sensor element 14 comprises a top portion 145 in which an activation sensor 144 is provided and which may be positioned squarely outside of the inhaler housing 124, and an elongated portion 148 extending into the flow channel 126, where the flow sensor 146 is provided at the far end so as to be positioned in the flow channel 126.

Again, the sensor element may be detachably attached to the inhaler, and in this situation to the canister end. The top portion 145 does not prevent the compression of the canister but is able to sense the force exerted to compress the canister. At least when the canister is depressed, the flow sensor 146 is provided in the flow channel 126 in order to sense or estimate the flow therein.

In figure 4, the sensor element 114 is illustrated. Illustrated is also a circuit or electronics 16 which may be the above-mentioned controller, storage, additional sensor, and/or communication means or the like.

In figure 4, a second flow channel 146' is illustrated through which air is drawn when air is drawn through the flow channel 126. The flow sensor 146 is positioned so as to sense air flow in the second flow channel 146'. The flow sensor 146 may be positioned at any position (two of which are illustrated) in the second flow channel 146, such as at the top and close to the electronics 16.

## Claims

1. An assembly (10) of:
- a medication inhaler (12) comprising:
- an inhaler housing (124) defining a first flow channel (126)
- a medication dispensing opening (128) into or in the first flow channel, and
- a manual activation element configured to cause medication to be dispensed into the first flow channel through the medication dispensing opening,
- a sensing element (14) comprising:
- an activation detector (144) configured to detect activation of the manual activation element, and
- a flow sensor (146), and
- a processor (16) in communication with the flow sensor and the activation detector, the processor being configured to detect an inhalation when a flow is sensed and an activation is detected,
wherein the sensing element is detachably attachable to the medication inhaler and **characterized in that** the sensing element comprises a portion (148) extending from the activation detector and into the first flow channel, a second flow channel (146') extending from the flow sensor to the first flow channel through the portion.

2. An assembly according to claim 1, wherein the medication inhaler comprises a compressible container (122) with pressurized liquid/fluid/gas positioned in the housing, where the first flow channel extends in the housing along the container and where the activation sensor is provided at one end of the container.

3. An assembly according to claim 1 or 2, wherein the flow sensor is provided at the activation detector.

4. An assembly according to any of the preceding claims, wherein the sensing element comprises a sensor housing portion (145) configured to engage the inhaler housing and form a part of the first flow channel, the flow sensor being provided in the sensor housing portion.

5. An assembly according to any of the preceding claims, wherein the processor forms part of the sensing element.

## Patentansprüche

1. Anordnung (10) von:
- einem medizinischen Inhalator (12), umfassend:
- ein Inhalatorgehäuse (124), das einen ersten Durchflusskanal (126) definiert,
- eine Medikationsausgabeöffnung (128) in den oder dem ersten Durchflusskanal, und
- ein manuelles Aktivierungselement, das konfiguriert ist, um zu bewirken, dass die Medikation durch die Medikationsausgabeöffnung in den ersten Durchflusskanal ausgegeben wird.
- ein Sensorelement (14), umfassend:
- einen Aktivierungsdetektor (144), der konfiguriert ist, um die Aktivierung des manuellen Aktivierungselementes zu erfassen, und
- einen Durchflusssensor (146), und
- einen Prozessor (16) in Kommunikation mit dem Durchflusssensor und dem Aktivierungsdetektor, wobei der Prozessor konfiguriert ist, um eine Inhalation zu erfassen, wenn ein Durchfluss gemessen wird und eine Aktivierung erfasst wird,
wobei das Sensorelement lösbar an dem Medikationsinhalator anbringbar ist und **dadurch gekennzeichnet ist, dass** das Sensorelement einen Abschnitt (148) umfasst, der sich vom Aktivierungsdetektor und in den ersten Durchflusskanal erstreckt, wobei sich ein zweiter Durchflusskanal (146') vom Durchflusssensor durch den Abschnitt zum ersten Durchflusskanal erstreckt.

2. Anordnung nach Anspruch 1, wobei der Medikationsinhalator einen komprimierbaren Behälter (122) mit unter Druck stehender Flüssigkeit/Flüssigkeit/Gas umfasst, die im Gehäuse positioniert sind, wobei sich der erste Durchflusskanal im Gehäuse entlang des Behälters erstreckt und wobei der Aktivierungssensor an einem Ende des Behälters bereitgestellt ist.

3. Anordnung nach Anspruch 1 oder 2, wobei der Durchflusssensor am Aktivierungsdetektor vorgesehen ist.

4. Anordnung nach einem der vorhergehenden Ansprüche, wobei das Sensorelement einen Sensorgehäuseabschnitt (145) umfasst, der konfiguriert ist, um in das Inhalatorgehäuse einzugreifen und einen Teil des ersten Durchflusskanals zu bilden, wobei der Durchflusssensor in dem Sensorgehäuseabschnitt bereitgestellt ist.

5. Anordnung nach einem der vorhergehenden Ansprüche, wobei der Prozessor einen Teil des Sensorelements bildet.

## Revendications

1. Montage (10) d'un :
- inhalateur de médicament (12) comprenant :
- un logement d'inhalateur (124) définissant un premier canal d'écoulement (126),
- une ouverture de distribution de médicament (128) jusque dans ou dans le premier canal d'écoulement, et
- un élément d'activation manuel configuré pour faire en sorte que le médicament soit distribué jusque dans le premier canal d'écoulement par l'ouverture de distribution de médicament,
- un élément de capteur (14) comprenant :
- un détecteur d'activation (144) configuré pour détecter l'activation de l'élément d'activation manuelle, et
- un capteur d'écoulement (146), et
- un processeur (16) en communication avec le capteur d'écoulement et le détecteur d'activation, le processeur étant configuré pour détecter une inhalation lorsqu'un écoulement est détecté et qu'une activation est détectée,
dans lequel l'élément de capteur est fixé de manière amovible à l'inhalateur de médicament,
**caractérisé en ce que** l'élément de détection comprend une partie (148) s'étendant du détecteur d'activation et jusque dans le premier canal d'écoulement, un second canal d'écoulement (146') s'étendant du capteur d'écoulement au premier canal d'écoulement à travers la partie.

2. Montage selon la revendication 1, dans lequel l'inhalateur de médicament comprend un récipient compressible (122) avec du liquide/fluide/gaz pressurisé positionné dans le logement, dans lequel le premier canal d'écoulement s'étend dans le logement le long du récipient et dans lequel le capteur d'activation est prévu à une extrémité du récipient.

3. Montage selon la revendication 1 ou 2, dans lequel le capteur d'écoulement est prévu sur le détecteur d'activation.

4. Montage selon l'une quelconque des revendications précédentes, dans lequel l'élément de détection comprend une partie de logement de capteur (145) configurée pour engager le logement d'inhalateur et former une partie du premier canal d'écoulement, le capteur d'écoulement étant prévu dans la partie de logement de capteur.

5. Montage selon l'une quelconque des revendications précédentes, dans lequel le processeur fait partie de l'élément de détection.
